# EUROPÄISCHE PATENTSCHRIFT

(11) **EP 3 798 295 B1**
(45) Veröffentlichungstag und Bekanntmachung des Hinweises auf die Patenterteilung: **20.12.2023**
(21) Anmeldenummer: 20180364.0
(22) Anmeldetag: 16.06.2020
(51) Int. Cl.: C11D 11/00, C11D 17/04

(54) **VERFAHREN ZUR HERSTELLUNG TENSID ENTHALTENDER ZUSAMMENSETZUNGEN IN EINEM SEQUENZIELLEN VERFAHREN**
METHOD FOR THE PREPARATION OF SURFACTANT-CONTAINING COMPOSITIONS IN A SEQUENTIAL METHOD
PROCÉDÉ DE FABRICATION DE COMPOSITIONS CONTENANT DES TENSIDES DANS UN PROCESSUS SÉQUENTIEL

(30) Priorität: 27.09.2019 DE 102019126124
(43) Veröffentlichungstag der Anmeldung: 31.03.2021
(73) Patentinhaber: Henkel AG & Co. KGaA, 40589 Düsseldorf (DE)
(72) Erfinder: Boesemann, Gerd, 40595 Düsseldorf (DE); Sunder, Matthias, 40593 Düsseldorf (DE); Blank, Volker, 51375 Leverkusen (DE)

(56) Entgegenhaltungen:
- EP-A1- 1 255 807
- DE-A1-102013 205 079
- DE-A1-102014 225 145
- DE-A1-102015 212 131
- DE-A1-102017 220 084

## Beschreibung

Die vorliegende Erfindung betrifft ein Verfahren zur Herstellung flüssiger Tensid enthaltender Zusammensetzungen in einem sequentiellen, kontinuierlichen Verfahren, welches es ermöglicht Mehrkammerbehältnisse gleichzeitig mit unterschiedlichen Zusammensetzungen zu füllen.

Flüssige, Tensid-enthaltende Zusammensetzungen sind aus dem Alltag nicht mehr wegzudenken. Einerseits handelt es sich hierbei um Körperpflegeprodukte, wie beispielsweise Shampoos, Duschgele oder Schaumbäder. Aber auch Wasch- oder Reinigungsmittel, wie Haushaltsreiniger, Weichspüler, Waschmittel für Wäsche, Bodenpflegemittel, Allesreiniger, manuelle Geschirrspülmittel, maschinelle Geschirrspülmittel oder Vollwaschmittel sind hiervon umfasst.

Ein Großteil dieser Zusammensetzungen wird heutzutage in einem Chargenprozess hergestellt. Der Chargenprozess, oft auch als Batchproduktion bezeichnet, ist ein diskontinuierliches Produktionsverfahren. Hier werden bestimmte Mengen von Einsatzstoffen gemäß einer vorgegebenen Rezeptur in einen Behälter gefördert und dort vermischt. Das Fassungsvermögen des Produktionsgefäßes, in welchem alle Bestandteile miteinander vermischt werden, begrenzt die Materialmenge, die in einer Charge oder einem Batch hergestellt wird.

In einem typischen Chargenprozess oder Batchprozess wird zunächst ein Reaktionsgefäß vollständig mit den Ausgangsstoffen, also den Edukten befüllt. Die Reaktion der Edukte miteinander hin zum Endprodukt erfolgt innerhalb des Reaktionsgefäßes. Ist die gegebenenfalls stattfindende Reaktion abgeschlossen, wird das Reaktionsgefäß vollständig entleert und die gewünschte Formulierung in geeignete Behälter zum Verkauf oder gegebenenfalls zur Lagerung gefüllt. Anschließend muss das Reaktionsgefäß für die nächste Befüllung vorbereitet werden. Dies bedeutet, insofern eine andere Formulierung hergestellt wird, eine gründliche Reinigung des Reaktionsgefäßes sowie gegebenenfalls der Leitungen, über welche die Ausgangsprodukte in das Reaktionsgefäß eingeführt werden.

Ein solcher Chargenprozess hat den Vorteil, dass die Formulierung der Rezeptur noch im Reaktionsgefäß bei Bedarf angepasst werden kann. Nachdosierungen einzelner Komponenten sind hier möglich. Unter Qualitätsaspekten ist hierzu berücksichtigen, dass die Möglichkeit der Chargenrückverfolgung besteht.

Nachteilig ist jedoch der große Platzbedarf. Ein Reaktionsgefäß wird immer vollständig gefüllt; das heißt, es werden immer große Mengen eines Produktes hergestellt. Ist eine Charge (oder ein Batch) hergestellt, muss sie zuerst verarbeitet werden, bevor ein weiterer Ansatz einer neuen Charge erfolgen kann. Ist eine direkte Weiterverarbeitung beziehungsweise Abfüllung nicht möglich, muss ein bereits hergestelltes Produkt außerhalb des Reaktionsgefäßes gelagert werden. Auch dies führt erneut zu einem hohen Platzbedarf sowie zur Entstehung weiterer Kosten.

Weiterhin erfordert der Wechsel in der Produktion von einem Produkt hin zu einem anderen einen großen Aufwand. Wird beispielsweise in einem ersten Chargenprozess ein Produkt hergestellt, welches einen bestimmten Farb- und einen bestimmten Geruchsstoff aufweist, so muss, bevor ein zweites Produkt mit einem unterschiedlichen Farb- und Geruchsprofil hergestellt wird, das Reaktionsgefäß sowie alle Zuleitungen gründlichst gereinigt werden, damit eine Verunreinigung der Chargen vermieden wird.

Neben dem diskontinuierlichen Chargenprozess sind auch kontinuierliche Verfahren zur Herstellung flüssiger, Tensid-enthaltender Zusammensetzungen bekannt. Kontinuierliche Prozesse bieten bessere Möglichkeiten zur Just-in-time-Produktion. Allerdings ist hier eine aufwendige Steuerung der einzelnen Prozessschritte notwendig. Im kontinuierlichen Prozess erfolgt die Durchmischung mittels statischer oder dynamischer Mischvorrichtungen und nicht in einem Reaktionsgefäß wie im Chargenprozess. Vielmehr erfolgt die Durchmischung innerhalb einer Leitung. In diese Leitung werden die einzelnen Zutaten einer Rezeptur in einer vordefinierten Reihenfolge eindosiert. Am Ende dieser Leitung erfolgt unmittelbar die Abfüllung. Ein Nachdosieren oder Ändern der Konzentration einzelner Bestandteile ist hier nicht möglich. Es ist eine gezielte und kontrollierte Überwachung der Zugabe jedes einzelnen Bestandteils notwendig.

Bei der Herstellung von Körperpflege-, Wasch- oder Reinigungsmitteln ist zudem zu beachten, dass die Zugabe von festen Bestandteilen notwendig sein kann. Diese können jedoch ausschließlich in einem Chargenprozess zugegeben werden. Die Zugabe fester Bestandteile in einem kontinuierlichen Verfahren ist nicht oder nur sehr schwer möglich. In kontinuierlichen Verfahren können nur flüssige Komponenten dosiert werden. Feste Bestandteile müsse in Form einer Aufschlämmung vorliegen, um dosiert werden zu können. Dies sorgt jedoch für Ungenauigkeiten in der Konzentration.

Die Zugabe fester Additive zu entsprechenden Zusammensetzungen gehört jedoch heute zum Stand der Technik. Feststoffe in Flüssigkeiten stabil zu suspendieren ist häufig problematisch, insbesondere wenn sich die Feststoffe bezüglich der Dichte von der Flüssigkeit unterscheiden, neigen sie dazu zu sedimentieren oder aufzuschwimmen. Auch die Einarbeitung von bestimmten Wirkstoffen (beispielsweise Bleichmittel Enzyme, Parfüme, Farbstoffe usw.) in flüssige Wasch- und Reinigungsmittel kann zu Problemen führen. Beispielsweise können Unverträglichkeiten zwischen den einzelnen Wirkstoffkomponenten der flüssigen Wasch- und Reinigungsmittel auftreten. Dies kann zu unerwünschten Verfärbungen, Agglomerationen, Geruchsproblemen und Zerstörung von waschaktiven Wirkstoffen führen.

Verfahren zur Herstellung von flüssigen Zusammensetzungen, die sich mit diesem Problem beschäftigen, sind beispielsweise in WO 2016/091733 A1 oder WO 2017/001218 A1 beschrieben.

Die DE 102013205079A1 und WO2001/60966 A1 beschreiben lösliche Mehrkammerbeutel für Waschmittel.

In der DE102017220084 A1 wird ein kontinuierliches Verfahren zur Herstellung Tensid-haltiger Flüssigkeiten offenbart.

Um Unverträglichkeiten zwischen Wirkstoffkomponenten zu vermeiden, werden flüssige Wasch- und Reinigungsmittel zunehmend in Mehrkammer-Behältnissen angeboten. In sogenannten Pouches oder Beuteln werden in zwei oder mehreren Kammern unterschiedliche Zusammensetzungen bereitgestellt. Dies ermöglicht es, Wechselwirkungen zwischen einzelnen Komponenten zu verhindern, in dem diese Komponenten in getrennten Kammern vorliegen. Bei der Benutzung werden die Zusammensetzungen dann freigesetzt. Dies ermöglicht eine längere Lagerung und eine größere Möglichkeit an Zusammensetzungen, da auf Unverträglichkeiten zwischen den einzelnen Wirkstoffkomponenten durch getrennte Bereitstellung eingegangen werden kann.

Die Bereitstellung in Mehrkammerbeuteln erfordert jedoch, dass die unterschiedlichen Zusammensetzungen gleichzeitig bereitgestellt werden, um diese dann in die jeweiligen Kammern der Mehrkammerbeutel abzufüllen. Eine Möglichkeit ist, die unterschiedlichen Zusammensetzungen in einem Chargenprozess getrennt voneinander herzustellen. Alternativ wäre es möglich, Verfahren, wie in WO 2017/001218 A1 oder WO 2016/091733 A1 beschrieben, einzusetzen, um die einzelnen Zusammensetzungen herzustellen. In beiden Fällen müsste dann jede Zusammensetzung hergestellt und gelagert werden, bis alle Zusammensetzung hergestellt sind. Anschließend erfolgt dann die Abfüllung. Bei einem Verfahren wie in WO 2017/001218 A1 beschrieben, könnten zwei oder mehr Verfahren parallel betrieben werden, um die einzelnen Zusammensetzungen herzustellen. Diese Möglichkeiten erfordern jedoch eine redundante Installation aller Komponenten und daraus resultierend einen hohen Platzbedarf. Zudem sind die benötigten Mengen der einzelnen Zusammensetzungen gering, da die Menge je Zusammensetzung in einer Kammer klein ist.

Entsprechend wäre die optimale Auslastung einer Anlage nicht gegeben, so dass auch Bedarf an einer wirtschaftlichen Lösung besteht.

Überraschenderweise hat sich gezeigt, dass die sich aus dem Stand der Technik ergebenden Nachteile durch ein Verfahren gemäß Anspruch 1, 2 oder 3 im Wesentlichen vermieden werden können. In einer weiteren Ausführungsform betrifft die Erfindung Mehrkammerbeutel gemäß Anspruch 15 sowie ein Verfahren zur Reinigung textiler Gebilde gemäß Anspruch 16.

In einer ersten Ausführungsform betrifft die vorliegende Erfindung somit ein Verfahren zur kontinuierlichen Herstellung wenigstens zwei voneinander verschiedener flüssiger, Tensid enthaltender Zusammensetzungen wie in den Ansprüche 1, 2 und 3 ausgeführt.

Die Lagerung erfolgt erfindungsgemäß nur für einen sehr kurzen Zeitraum, der sicherstellt, dass von der ersten Zusammensetzung und der zweiten Zusammensetzung ausreichende Mengen vorhanden sind, um diese gleichzeitig in getrennte Kammern eines Mehrkammerbehältnisse abzufüllen. Überraschenderweise hat sich gezeigt, dass eine entsprechende sequenzielle Herstellmethode von wenigstens zwei voneinander verschiedenen Zusammensetzungen es ermöglicht, mit hoher Präzision Zusammensetzungen bereitzustellen, die gleichzeitig in einen Mehrkammerbeutel abgefüllt können, ohne dass Platzbedarf für parallel betriebene Anlagen besteht.

Es wäre auch denkbar eine Vorrichtung, wie in WO 2017/001218 A1 beschrieben zu verwenden und den Endstrom in 2, 3 oder mehr Ströme voreinander aufzutrennen. Es hat sich jedoch gezeigt, dass eine solche Variante mit einem Splitter technisch nicht derart realisierbar ist, dass drei gleich große Teilströme erhalten werden, so dass Schwankungen in der Rezeptur der einzelnen Zusammensetzungen nicht zu vermeiden wären. Dies soll jedoch vermieden werden, da eine gleichbleibende Zusammensetzung notwendig ist, um für jeden Mehrkammerbeutel die gleiche Reinigungsleistung sicherstellen zu können.

Das erfindungsgemäße Verfahren stellt damit ein kontinuierliches Herstellungsverfahren für zwei oder mehr voneinander verschiedene flüssige Tensid enthaltende Zusammensetzungen dar, wobei sich die Zusammensetzungen in mindestens einer Komponente unterscheiden. Entsprechend wird zunächst eine Grundmischung bereitgestellt, in welcher die Bestandteile enthalten sind, die in beiden Zusammensetzungen enthalten sind, wie beispielsweise Tensid und Lösungsmittel.

Die Zusammensetzungen werden dann dadurch erhalten, dass die Grundmischung mit einem ersten Additiv versetzt wird, um eine erste Zusammensetzung zu erhalten, und mit einem zweiten Additivversetzt wird, um eine zweite Zusammensetzung zu erhalten. Das mindestens eine erste und das mindestens eine zweite Additiv sind voneinander verschieden. In einer ersten Ausführungsform des Verfahrens umfassen sowohl das mindestens eine erste Additiv als auch das mindestens eine zweite Additiv wenigstens einen Farbstoff. Dabei sind die Farbstoffe voneinander verschieden, so dass die Zusammensetzungen sich zumindest in ihrer Farbe voneinander unterscheiden. Geeignete Farbstoffe, mit welchen eine flüssige Tensid enthaltende Zusammensetzung eingefärbt werden kann, ohne dass dies in der Anwendung nachteilig ist und beispielsweise zur Verfärbung von Textilien oder anderen Oberflächen führt, mit welchen die Zusammensetzungen in Kontakt kommen, sind dem Fachmann hinlänglich bekannt.

Vorzugsweise werden in dem erfindungsgemäßen Verfahren nicht nur zwei, sondern drei, vier oder mehr voneinander verschiedene Zusammensetzungen hergestellt. Entsprechend durchläuft im Falle von drei voneinander verschiedenen Zusammensetzungen die Grundmischung zyklisch mindestens die folgenden Schritte:
a. versetzen des Stroms der Grundmischung für eine erste Zeitdauer mit mindestens einem ersten Additiv, um eine erste Zusammensetzung zu erhalten,
   und
b. versetzen des Stroms der Grundmischung für eine zweite Zeitdauer mit mindestens einem zweiten Additiv, um eine zweite Zusammensetzung zu erhalten,
c. versetzen des Stroms der Grundmischung für eine dritte Zeitdauer mit mindestens einem dritten Additiv, um eine dritte Zusammensetzung zu erhalten,
wobei das mindestens eine erste Additiv und das mindestens eine zweite Additiv und das mindestens eine dritte Additiv voneinander verschieden sind.

Entsprechend sind in einem solchen Verfahren das mindestens eine erste Additiv, das mindestens eine zweite Additiv und das mindestens eine dritte Additiv voneinander verschieden.

Analog durchläuft die Grundmischung zyklisch mindestens die folgenden Schritte für den Fall, dass vier voneinander verschiedene flüssige, Tensid enthaltende Zusammensetzungen hergestellt werden:
a. versetzen des Stroms der Grundmischung für eine erste Zeitdauer mit mindestens einem ersten Additiv, um eine erste Zusammensetzung zu erhalten,
   und
b. versetzen des Stroms der Grundmischung für eine zweite Zeitdauer mit mindestens einem zweiten Additiv, um eine zweite Zusammensetzung zu erhalten,
c. versetzen des Stroms der Grundmischung für eine dritte Zeitdauer mit mindestens einem dritten Additiv, um eine dritte Zusammensetzung zu erhalten,
d. versetzen des Stroms der Grundmischung für eine vierte Zeitdauer mit mindestens einem vierten Additiv, um eine vierte Zusammensetzung zu erhalten.

Auch hier sind das mindestens eine erste Additiv, das mindestens eine zweite Additiv und das mindestens eine dritte Additiv ebenso wie das mindestens eine vierte Additiv voneinander verschieden.

Erste, zweite, ggf. dritte und ggf. vierte sowie jede weitere Zeitdauer können gleich oder voneinander verschieden sein. Die Zeitdauer kann auch wechseln. Durchläuft die Grundmischung beispielsweise zyklisch die Schritte A und B, so kann bei einem ersten Durchlauf des Schrittes A die erste Zeitdauer t1 betragen, die zweite Zeitdauer für Schritt B beträgt t2. Bei der zyklischen Wiederholung der Schritte würde dann die erste Zeitdauer t3 betragen und die zweite Zeitdauer t4. Weitere zyklische Wiederholungen sind möglich. Dabei können die Zeitdauern t1 und t3 beispielsweise voneinander verschieden sein. Sie können auch voneinander verschieden sein. Es kann t3 länger oder kürzer als t1 sein. In einem erneuten zyklischen Durchlauf wäre die erste Zeitdauer t5 und die zweite Zeitdauer t6. So kann beispielsweise t3 größer als t1 sein und t5 könnte t1 entsprechen. Es ist auch möglich, dass t5 kleiner t3 aber größer t1 ist. Auch könnte t5 kleiner t1 sein oder größer t3. Es wäre auch möglich, dass t5 t3 entspricht.

Für die zweite Zeitdauer t2, t4 und t6 sind ebenfalls alle Variationen möglich. Die Einstellung der Zeitdauer ist von der Menge an Zusammensetzung, die hergestellt wird, um einzelnen Kammern von Mehrkammerbeuteln zu befüllen, abhängig. Die Größe der Kammern kann variieren, so dass der Mengenbedarf an Zusammensetzungen, die bereitgestellt werden variiert. Der Fachmann ist hier in der Lage, die Zeitdauern zu optimieren, um die Zwischenlagerung vor der Abfüllung zu optimieren.

Entsprechendes gilt natürlich nicht nur für die erste und zweite Zeitdauer, sondern auch die dritte und jede weitere Zeitdauer, abhängig davon wie viele voneinander unterschiedliche Zusammensetzungen bereitgestellt werden.

Das mindestens eine erste Additiv und/oder das mindestens eine zweite Additiv und/oder ggf. das mindestens eine dritte und ggf. jedes weitere Additiv weisen vorzugsweise neben dem Farbstoff weiterhin wenigstens ein oder mehrere Enzyme auf, wobei die Enzyme insbesondere bevorzugt voneinander verschieden sind.

Es ist auch möglich, dass das mindestens eine erste Additiv und/oder das mindestens eine zweite Additiv und/oder das ggf. vorhandene mindestens eine dritte Additiv und/oder jedes ggf. weiter vorhandene Additiv wenigstens einen optischen Aufheller aufweisen. Weist ein Additiv einen optischen Aufheller auf, so enthält dies kein Enzym. Vorzugsweise umfasst wenigstens eines der Additive mindestens ein Enzym und wenigstens eines der Additive mindestens einen optischen Aufheller. Optische Aufheller (sogenannte "Weißtöner") können den flüssigen Wasch- und Reinigungsmitteln zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen im Sinne der vorliegenden Erfindung stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate. Die optischen Aufheller werden vorzugsweise in Mengen zwischen 0,03 und 0,3 Gew.-%, bezogen auf das fertige Mittel, eingesetzt.

Die Grundmischung, die wenigstens ein Tensid und wenigstens ein Lösungsmittel aufweist, kann in einem kontinuierlichen Verfahren oder einem Batch Verfahren hergestellt werden. Vorzugsweise wird die Grundmischung dadurch bereitgestellt, dass in einer Vormischung, die wenigstens ein Tensid und wenigstens ein Lösungsmittel aufweist, hergestellt wird, zu welcher dann in einem kontinuierlichen Verfahren wenigstens eine weitere Komponente zugegeben wird. Diese eine weitere Komponente können beispielsweise Parfüme, Lösungsmittel, Co-Tenside, Konservierungsmittel, Farbschutzmittel, Bleichinhibitoren, pH-Stellmittel, Strukturhilfsmittel, Korrosionsinhibitoren und andere sein. Dabei sind diese Komponenten derart ausgewählt, dass sie in allen hergestellten Zusammensetzungen enthalten sind.

In dem bevorzugten Verfahren, in welchem in einem Batch Verfahren eine Vormischung hergestellt wird, zu welcher dann in einem kontinuierlichen Verfahren wenigstens eine weitere Komponente zugegeben wird, beträgt der Anteil aller Bestandteile der im Batch verfahren hergestellten Mischung 1 Gew.-% bis 99 Gew.-%, bevorzugt 5 Gew.-% bis 95 Gew.-%, insbesondere 20 Gew.-% bis 90 Gew.-%, bezogen auf das Gesamtgewicht der Grundmischung. Der Anteil aller Bestandteile, die im kontinuierlichen Verfahren eingebracht werden, liegt vorzugsweise bei 1 Gew.-% bis 99 Gew.-%, insbesondere 5 Gew.-% bis 95 Gew.-%, bevorzugt 10 Gew.-% bis 80 Gew.-%.

In diesem bevorzugten Verfahren weist die Vormischung eine Temperatur im Bereich von 35° C oder mehr auf. Dies bedeutet, dass die Vormischung, die aus dem Batch Kessel eingespeist wird und dann in einer kontinuierlichen Anlage weiterverarbeitet wird, beim Eintritt in die kontinuierliche Anlage eine Temperatur von 35° C oder mehr aufweist. Die Temperatur der Mischung wird im Batch-Kessel und in der Konti-Anlage an der Zuleitung mit einem handelsübliches PT100 Widerstandsthermometer bestimmt. Im Batch-Kessel wird das Thermometer neben dem Auslass montiert, über den die Mischung in die Konti-Anlage gelangt. Üblicherweise weist die Mischung im Batch-Kessel beim Ausleiten die gleiche Temperatur auf, wie zum Zeitpunkt des Einleitens in die Konti-Anlage. Geprüft wird dies über ein zweites PT100 Widerstandsthermometer, das in der Konti-Anlage an der Stelle montiert ist, an der die Zuleitung der Mischung erfolgt. Es wird immer vermieden, dass die Mischung zwischen dem Batch-Kessel und der Einleitung in die Konti-Anlage unter 35 °C abkühlt. Ggfs. wird die Temperatur der Mischung im Batch-Kessel deutlich über 35 °C eingestellt, um die Mischung mit 35 °C oder mehr in die Konti-Anlage einzuleiten. Die Mischung wird also zwischen Kessel und Konti-Anlage nicht wieder erwärmt, bevor sie in die Konti-Anlage gelangt. Vielmehr wird die Wärme der Batch-Mischung ausgenutzt, um die Mischung ohne weitere Aufwärmung mit einer Temperatur von 35 °C oder mehr in die Konti-Anlage einzuspeisen. Dies ist ein besonderer Vorteil der vorliegenden Erfindung, da er zur Energieeinsparung und zur Stabilisierung der Mischung beiträgt.

Üblicherweise werden Vormischungen im Batchverfahren bei einer erhöhten Temperatur hergestellt. Diese liegt bei den meisten Verfahren bei 35°C oder darüber. Häufig weist die Vormischung am Ende des Batchverfahrens Temperaturen im Bereich vom 40°C bis 90°C auf.

Die Batch-Temperatur basiert häufig darauf, dass ein Lösungsmittel mit einer Temperatur von 40 °C oder mehr, insbesondere von 50 °C oder mehr, bevorzugt von 60 °C oder mehr eingesetzt wird. Diese Temperaturen ermöglichen, dass die Aktivstoffe, welche im Lösungsmittel im Batchverfahren gelöst werden sollen, sich gut auflösen oder darin verteilen. Das Lösungsmittel kann erfindungsgemäß mit einer gegenüber Raumtemperatur erhöhten Temperatur in das Batchverfahren eingebracht werden. Raumtemperatur im Sinne der vorliegenden Erfindung meint 20 °C. Neben dem Lösungsmittel können auch andere Substanzen in den Batch gegeben werden, die eine der oben beschriebenen Temperaturen aufweisen.

Es ist jedoch auch möglich, dass die gesamte Vormischung im Batchverfahren erwärmt wird. Dies kann einerseits durch Reibungs- oder Scherkräfte, die bei der Durchmischung im Batchverfahren auftreten, erfolgen. Heizelemente können ebenfalls zum Heizen der Vormischung im Batch-Kessel zum Einsatz kommen. Im Batchverfahren laufen jedoch auch häufig exotherme Reaktionen ab, bei denen zusätzliche Wärme frei gegeben wird, wodurch die Temperatur im Rührkessel des Batchverfahrens ansteigt. Entsprechende exotherme Reaktionen sind beispielsweise Neutralisationsreaktionen, die auftreten, wenn Tenside, insbesondere Aniontenside, im Batch durch Neutralisation der korrespondierenden Säure hergestellt werden wenn beispielsweise von konzentrierte Lauge verdünnt wird.

So können Säuren der Aniontenside, die hierin offenbart werden, mit einem geeigneten Neutralisationsmittel im Batchkessel oder außerhalb des Batchkessels neutralisiert werden. Die durch Ablauf der Neutralisationsreaktion im Kessel oder durch die Zuleitung des warmen Neutralisats im Kessel entstehende Wärme, erhöht die Temperatur der Mischung im Batch. Dies verbessert die Löslichkeit der einzelnen Komponenten in der Mischung. Als Neutralisationsmittel im Rahmen der vorliegenden Erfindung sind sämtliche Substanzen geeignet, die in der Lage sind, das Aniontensid in seiner Säureform zu neutralisieren, d.h. in ein Aniontensidsäuresalz zu überführen.

Das Neutralisationsmittel kann in flüssigem oder festem Zustand hinzugegeben werden. Neutralisationsmittel im flüssigen Zustand schließt Lösungen und Suspensionen fester Neutralisationsmittel ein. So kommen beispielsweise Alkalihydroxide wie NaOH oder KOH, basische Oxide wie Alkalimetalloxide oder basische Salze wie beispielsweise Carbonate in Betracht.

Weitere Neutralisationsmittel sind Ammoniak und Amine. Vorzugsweise werden Amine gewählt, insbesondere aus der Gruppe bestehend aus Monoethanolamin, Trimethylamin, Triethylamin, Tripropylamin, Triethanolamin, N-Methylmorpholin, Morpholin, 2,2-Dimethylmonoethanolamin, N,N-Dimethylmonoethanolamin und Mischungen davon. Ganz besonders bevorzugt werden die Amine, da sie gut handhabbar sind und bei der Neutralisation kein Wasser entsteht. Als besonders bevorzugt gilt Monoethanolamin.

Die Neutralisationsmittel können mit den für Wasch-, Reinigungs- und Pflegemitteln üblichen Aniontensidsäuren kombiniert werden, insbesondere mit den hierin offenbarten Aniontensiden korrespondierenden Aniontensidsäuren. Neutralisationsmittel werden vorzugsweise in einem bestimmten molaren stöchiometrischen Verhältnis zur Aniontensidsäure eingesetzt, das unter den gewählten Reaktionsbedingungen den vollständigen Ablauf der Reaktion erlaubt. Beispiels weise kann das molare Verhältnis Neutralisationsmittel zu Aniontensidsäure 0,5 : 1 bis 10 : 1, vorzugsweise 1 : 1 bis 3: 1 betragen.

Es kann vorteilhaft sein, die Aniontensidsäure und/oder das Neutralisationsmittel oder die Mischung im Batch-Kessel zu erhitzen, um den Start der Neutralisation zu beschleunigen. Als besonders bevorzugte Aniontensidsäure gilt C9 bis C13 Alkylbenzolsulfonsäure, insbesondere lineare C9 bis C13 Alkylbenzolsulfonsäure. Insbesondere lineare C9 bis C13 Alkylbenzolsulfonsäure (LAS-Säure oder HLAS) und Monoethanolamine, welche vorzugsweise Bestandteile der im Batchverfahren hergestellten Vormischung oder der Grundmischung sind, reagieren unter Wärmeentwicklung miteinander. In einer bevorzugten Ausführungsform des erfindungsgemäßen Verfahrens erfolgt im Batchverfahren eine Neutralisation von lineare C9- C13 Alkylbenzolsulfonsäure mit Monoethanolamin.

Besonderer Vorteil der Verwendung von Monoethanolamin ist die Vermeidung der Bildung von Wasser als Neutralisationsprodukt. Insbesondere bei der Herstellung von wasserfreien oder wasserarmen Mischungen und Zusammensetzung ist dies bedeutsam. Es ist vorteilhaft, die Aniontenside erst im Batch aus den korrespondierenden Säuren herzustellen, da zum einen die Säure günstiger zu erwerben ist und die Neutralisationswärme die Mischung erwärmt, so dass die Auflösung der Komponenten in der Vormischung beschleunigt wird. In bestimmten Ausführungsformen kann auf die weitere gezielte Zufuhr von Wärme verzichtet werden, was einen ökonomischeren Prozessablauf erlaubt. Die Auch bei der Mischung einer oder mehrere Aktivsubstanzen in einem Lösungsmittel kann es zu einer Wärmefreisetzung kommen. Eine solche ist im Batchverfahren bevorzugt, da hierdurch die meisten Bestandteile im Lösungsmittel besser löslich sind.

Darüber hinaus ist es bekannt, dass wichtige Rohstoffe, wie z. B. Enzyme, Silicone (Entschäumer), Duftstoffe oder Lösungsmittel mit niedrigem Flammpunkt nur bei Temperaturen T < 30 °C in einer flüssigen Masse stabil bleiben oder dosiert werden können. Es besteht zudem eine hohe Wahrscheinlichkeit, dass bei T > 30 °C die Degradation von in der Zusammensetzung eventuell enthaltenen Enzymen deutlich schneller läuft und dadurch ein Abbau der Produktleistung verursacht wird. Ebenso kann bei erhöhten Temperaturen eine als Entschäumer enthaltene Silicon-Emulsion brechen und dadurch eine Phasentrennung im Produkt entstehen. Dies kann in einer Aufschäumung der Charge resultieren, so dass eine Weiterverarbeitung hier nicht mehr möglich ist. Erfindungsgemäß ist daher die im Batchverfahren hergestellte Mischung vorzugsweise frei von Entschäumern. Diese können erfindungsgemäß im kontinuierlichen Verfahren in die Zusammensetzung eingebracht werden. Daher kann in einer Ausführungsform die Vormischung im kontinuierlichen Verfahren mit Entschäumer versehen werden, insbesondere derart, dass die Zusammensetzung mindestens 0,01 Gew.-% Entschäumer aufweist. Im Batchverfahren können Lösungsmittel mit niedrigem Flammpunkt entweichen und hierdurch eine explosionsfähige Atmosphäre bilden, wodurch die Sicherheit der Herstellung gefährdet werden kann, so dass auch diese vorzugsweise im kontinuierlichen Verfahren zugegeben werden. Auch die Grundmischung kann unabhängig von dem Herstellverfahren Entschäumer aufweisen.

Enzyme im Sinne der vorliegenden Erfindung sind alle bekannten in Wasch- oder Reinigungsmittelverfahren geeignete Enzyme, z.B. Amylasen, Lipasen, Cellulasen, Pektinase und Proteasen.

Entschäumer im Sinne der vorliegenden Erfindung sind Silicone. Vorzugsweise beträgt die Konzentration der Entschäumer in der Zusammensetzung 0,005 bis 0,02 Gew.-%.

Besonders bevorzugt sind Siliconöle. Geeignete Silicone sind übliche Organopolysiloxane, die einen Gehalt an feinteiliger Kieselsäure, die wiederum auch silaniert sein kann, aufweisen können. Derartige Organopolysiloxane sind beispielsweise in der europäischen Patentanmeldung EP 0496510 A1 beschrieben. Besonders bevorzugt sind Polydiorganosiloxane, die aus dem Stand der Technik bekannt sind. In der Regel enthalten die Polydiorganosiloxane feinteilige Kieselsäure, die auch silaniert sein kann. Insbesondere geeignet sind kieselsäurehaltige Dimethylpolysiloxane.

Durch eine erfindungsgemäß bevorzugte Kühlung im kontinuierlichen Verfahren wird die Temperatur der Mischung reduziert. Vorzugsweise beträgt die Temperatur der Grundmischung am Ende des kontinuierlichen Verfahrens vor dem zyklischen Durchlauf der Schritte a) und b) unter 35°C, insbesondere 25°C, oder weniger.

Eine Kühlung der im Batchverfahren hergestellten Vormischung kann im kontinuierlichen Verfahren auf unterschiedliche Art und Weise durchgeführt werden. Eine Konti-Anlage, in der ein entsprechendes kontinuierliches Verfahren durchgeführt werden kann, umfasst eine Hauptleitung, in welcher die unterschiedlichen Bestandteile der erfindungsgemäßen Zusammensetzung in vorgegebener definierter Reihenfolge über Nebenleitungen eingebracht werden. Des Weiteren erfolgt eine Verdünnung der üblicherweise im Batchverfahren hergestellten hochkonzentrierten Vormischung mit einem geeigneten Lösungsmittel, üblicherweise Wasser. Eine Kühlung kann nun dadurch erfolgen, dass die zugeführten Komponenten sowie das Lösungsmittel eine geringere Temperatur als die der Vor-Mischung aufweisen. Des Weiteren ist es auch möglich, dass um das Hauptrohr, in welchem aufgrund der Fließeigenschaften eine Durchmischung stattfindet, entsprechende Kühlvorrichtungen angebracht sind. Eine Kühlung kann erfindungsgemäß direkt oder indirekt erfolgen. Als geeignete Apparate (Kühlvorrichtungen) sind Plattenwärmetauscher, Rohrbündelwärmetauscher, Doppelrohrwärmetauscher mit oder ohne Mischelement im produktseitigen Rohr zu nennen.

Um eine verbesserte Durchmischung zu ermöglichen, kann vorgesehen sein, in die Hauptleitung der Konti-Anlage statische und/oder dynamische Mischer einzubringen. Sind statische Mischer vorgesehen, so können auch diese als Kühlung wirken. Hierfür können die statischen Mischer entweder ein Material umfassen, welches gekühlt werden kann, wie beispielsweise ein Metall oder ein wärmeleitender Kunststoff. Es ist auch denkbar, dass durch den statischen Mischer ein geeignetes Kühlmittel fließt, wodurch eine Abkühlung der Mischung erfolgt.

Das kontinuierliche Verfahren ist dadurch gekennzeichnet, dass innerhalb der Anlage, in der das kontinuierliche Verfahren stattfindet, vorzugsweise ein Überdruck gegenüber dem Umgebungsdruck herrscht. Die Mischung wird dabei durch ein Leitungssystem geleitet. Mittels Pumpen werden die Fließgeschwindigkeit der Zusammensetzung und damit auch der Druck in dem Leitungssystem gesteuert. An dem Leitungssystem angebrachte Drucksensoren erlauben es, über eine Rückkopplung zu den Pumpen, den Druck innerhalb des Leitungssystems zu kontrollieren. Beispielsweise können Drucksensoren der Firma Endress und Hauser, Deutschland, zum Einsatz kommen. Die Hauptleitung, in welche die Mischung eingeleitet wird, beziehungsweise der darin fließende Materialstrom, wird Hauptstrom genannt. In diese Hauptleitung werden auch die weiteren Aktivstoffe oder Komponenten der Zusammensetzung dosiert. Das kontinuierliche Verfahren unter Überdruck erlaubt es zudem, einen Gas-/Lufteintrag in die Zusammensetzung zu vermeiden. Vorzugsweise wird das kontinuierliche Verfahren bei einem gegenüber dem Umgebungsdruck erhöhten Druck von 0,1 bis 6 bar, insbesondere von 0,5 bis 4 bar durchgeführt.

In diesem kontinuierlichen Verfahren werden in einer kontinuierlichen Anlage alle Stoffe in flüssiger Form in die Hauptleitung zusammen dosiert und mittels dynamischer und/oder statischer Mischer homogenisiert. Flüssige Produkte im Sinne der vorliegenden Erfindung sind Flüssigkeiten oder Lösungen von Feststoffen in einem geeigneten Lösungsmittel ebenso wie stabile Suspensionen, Dispersionen oder Emulsionen.

Ein Stoff, wie zum Beispiel eine Zusammensetzung oder Mischung, ist gemäß Definition der Erfindung flüssig, wenn sie bei 25 °C und 1013 mbar im flüssigen Aggregatzustand vorliegt. Ein Stoff ist erfindungsgemäß fest oder festförmig, wenn er bei 25 °C und 1013 mbar im festen Aggregatzustand vorliegt.

Die Begriffspaare Tensid/Tenside, Phosphonat/Phosphonate, Aniontensid/Aniontenside, Niotensid/Niotenside und ähnliche Begriffe sollen die identische Bedeutung haben und sowohl den Singular als auch den Plural erfassen.

Die Vormischung weist vorzugsweise eine hohe Konzentration an Tensid auf. Vorzugsweise handelt es sich hierbei um wenigstens ein Aniontensid. Die Vormischung weist vorzugsweise Aniontensid mit einem Anteil von 5 Gew.-% bis 40 Gew.-%, insbesondere von 8 Gew.-% bis 36 Gew.-%, besonders bevorzugt von 10 Gew.-% bis 30 Gew.-%, noch mehr bevorzugt von 20 Gew.-% bis 28 Gew.-% auf.

Weiter bevorzugt weist die Vormischung Niotensid mit einem Anteil von 1 Gew.-% bis 27 Gew.-%, insbesondere von 10 Gew.-% bis 26 Gew.-%, besonders bevorzugt von 15 Gew.-% bis 25 Gew.-% auf.

Aniontenside (anionische Tenside) und Niotenside (nicht ionische Tenside) lassen sich im Batchverfahren gut in ein geeignetes Lösungsmittel einarbeiten. Dies ermöglicht die Herstellung einer Vormischung mit einer hohen Konzentration an Tensiden, wobei die Vormischung dann im kontinuierlichen Verfahren je nach gewünschtem Endprodukt entsprechend verdünnt werden kann. Dies ermöglicht eine hohe Flexibilität in der Herstellung der gewünschten Zusammensetzung. Erfindungsgemäße im Batch-Verfahren hergestellte Grundmischungen, insbesondere Vormischungen, die wenigstens ein Tensid aufweisen (Tensidmischungen), sind üblicherweise nur bei erhöhter Temperatur stabil, so dass bevorzugt die im Batchverfahren hergestellte Mischung eine Temperatur von über 40 °C aufweist und solange sie diese Temperatur besitzt in das kontinuierliche Verfahren eingebracht wird. Dabei ist es wünschenswert, dass im kontinuierlichen Verfahren eine schnelle Verdünnung der Tensidmischung erfolgt, da es ansonsten zu einer Ausflockung der Tenside kommen kann. Neben den Tensiden kann es auch zu einer Ausflockung von Seifen oder Phosphonaten kommen. Bei bestimmten Tensiden würde bei einer langsamen Verdünnung eine Mischung sehr hoher Viskosität entstehen, die dann im Folgenden nicht mehr weiterverarbeitet werden könnte. Eine schnelle Verdünnung kann im kontinuierlichen Verfahren einfach ermöglicht werden, da die Dosierung der Mischung im Verhältnis zu einer Zudosierung von Wasser in die Hauptleitung gut zu kontrollieren ist. Besonders bevorzugt ist es, in der Konti-Anlage einen hochscherenden Mischer (Scherrate 1000 1/s bis 10000 1/s) für die Durchmischung, zum Beispiel einen sogenannten Pentax-Mischer, einzusetzen. Dadurch wird eine flexiblere Herstellung einer Ausgangsmischung im Batch möglich, da nun weniger Limitierungen hinsichtlich der Batch-Mischung vorliegen. Damit sind höher konzentrierte Mischungen möglich, die durch Verdünnung in einer Konti-Anlage flexibel differenziert werden können. Zudem kann der Lösungsmittel Anteil in der Batch-Mischung reduziert werden, weshalb ein kleinerer Batch-Kessel verwendet werden kann. Dies spart Investitions-, Reinigungs- und Instandhaltungskosten. Somit erlaubt es die vorliegende Erfindung, die Mittel kostengünstiger und effizienter herzustellen.

Unter dem Begriff Phosphonat werden hierin solche Phosphonate verstanden, die als Komplexbildner in den erfindungsgemäß hergestellten Zusammensetzungen wirken. Es gilt zu betonen, dass Komplexbildner wichtiger Bestandteil erfindungsgemäßer Zusammensetzungen sind. Daher ist es so vorteilhaft, nun Phosphonate in größeren Anteilen in Herstellverfahren einsetzen zu können.

In einer ganz besonders bevorzugten Ausführungsform weist die im Batchverfahren hergestellte Vormischung einen Gesamtgehalt an Phosphonat von 0,5 Gew.-% bis 8,0 Gew.-%, vorzugsweise von 1 ,0 Gew.-% bis 5 Gew.-%, noch weiter bevorzugt von 1 ,5 Gew.-% bis 3,0 Gew.-% auf. Die komplexbildenden Phosphonate umfassen neben der 1-Hydroxyethan-1,1-diphosphonsäure eine Reihe unterschiedlicher Verbindungen wie beispielsweise Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriamin-pentamethylen-phosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen.

Dementsprechend kann es, insbesondere wenn die Zusammensetzungen auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugt hergestellte Grundmischung, insbesondere die Vormischung, enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1 ,1-diphosphonsäure (HEDP) und/oder deren Salze;
e) 2-Phosphonobutan-1 ,2,4-tricarbonsäure (PBTC) und/oder deren Salze;
f) Hexamethylendiamintetra(methylenphosphonsäure) (HDTMP) und/oder deren Salze; g) Nitrilotri(methylenphosphonsäure) (NTMP) und/oder deren Salze.

Besonders bevorzugt werden Mischungen, welche als Phosphonate 1-Hydroxyethan-1 ,1-diphosphonsäure (HEDP) oder Diethylentriaminpenta(methylenphosphonsäure) (DTPMP) enthalten. Selbstverständlich können die erfindungsgemäßen Mischungen zwei oder mehr unterschiedliche Phosphonate enthalten. Bevorzugte erfindungsgemäße Mischungen sind dadurch gekennzeichnet, dass das Wasch- oder Reinigungsmittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht der Vor-Mischung vorzugsweise von 0,1 bis 8,0 Gew.-%, bevorzugt von 0,2 bis 5,0 Gew.-% und insbesondere von 0,5 bis 3,0 Gew.-% beträgt.

In einer weiteren bevorzugten Ausführungsform weist die im Batchverfahren hergestellte Vor-Mischung einen Gesamtgehalt an Fettsäure von 3,0 Gew.-% bis 20 Gew.-%, vorzugsweise von 5,0 Gew.-% bis 15 Gew.-%, noch weiter bevorzugt von 7,0 Gew.-% bis 10 Gew.-% auf.

Stabil im Sinne der vorliegenden Erfindung bedeutet, dass Aufrahmungen, Phasentrennung, Sedimentation, Ausflockungen oder Stippen, Wolken, Eintrübungen, ein milchiges Aussehen, Verfestigung oder Farb-Veränderungen nicht zu beobachten sind. Bevorzugt ist die im Batch-Verfahren hergestellte Mischung (Grundmischung) über einen Zeitraum von 1 Tag oder mehr, insbesondere von 5 Tagen oder mehr oder von 1 Woche oder mehr, bevorzugt von 2 Wochen oder mehr und insbesondere von 3 Wochen oder mehr, bevorzugt von 4 Wochen oder mehr stabil, wenn sie bei einer Temperatur von 40 °C oder darüber, insbesondere von 40 °C bis 90 °C gelagert wird. Bevorzugt ist die Grundmischung, wenn sie bei 40 °C gelagert wird, 2 Wochen oder länger, insbesondere 4 Wochen stabil. Für die Vormischung gelten dieselben Definitionen zur Stabilität.

Die erfindungsgemäß hergestellten Zusammensetzungen sind vorzugsweise über einen Zeitraum von 4 Wochen oder mehr, insbesondere von 8 Wochen oder mehr, bevorzugt von 12 Wochen oder mehr hinweg stabil. Dabei kann die Zusammensetzung bei Raumtemperatur oder einer darüber liegenden Temperatur gelagert werden, insbesondere bei 20 °C bis 40 °C. Besonders bevorzugt ist die Zusammensetzung bei einer Lagerung bei 40 °C über einen Zeitraum von wenigstens 12 Wochen hinweg stabil.

Die Zusammensetzungen können erfindungsgemäß ein oder mehrere Tenside aufweisen. Diese Tenside sind ausgewählt aus der Gruppe, die aus anionischen, kationischen, zwitterionischen, nichtionischen Tensiden sowie deren Mischungen besteht. Umfassen die Zusammensetzungen beziehungsweise die Grundmischung mehrere Tenside, so kann es sich hierbei beispielsweise um mehrere unterschiedliche nichtionische Tenside handelt. Es ist jedoch auch möglich, dass die Zusammensetzungen beziehungsweise die Grundmischung beispielsweise sowohl nichtionische als auch anionische Tenside umfasst. Analog gilt dies für die anderen Tenside. Vorzugsweise umfassen die Zusammensetzungen und/oder die Mischung wenigstens ein anionisches Tensid sowie wenigstens ein nichtionisches Tensid. Umfasst die Vor-Mischung ein oder mehrere Tenside, können bei Bedarf weitere Tenside im kontinuierlichen Verfahren zu dosiert werden.

Anionische Tenside sind bevorzugt ausgewählt aus der Gruppe bestehend aus C₉₋₁₃-Alkylben-zolsulfonaten, Olefinsulfonaten, C₁₂₋₁₈-Alkansulfonaten, Estersulfonaten, Alk(en)ylsulfaten, Fettalkohohlethersulfaten und Mischungen daraus. Es hat sich gezeigt, dass sich diese Sulfonat-und Sulfat-Tenside besonders gut zur Herstellung stabiler flüssiger Zusammensetzungen insbesondere solcher mit Fließgrenze eignen. Flüssige Zusammensetzungen, die als anionisches Tensid C₉₋₁₃-Alkylbenzolsulfonate und Fettalkoholethersulfate umfassen, weisen besonders gute, dispergierende Eigenschaften auf. Als Tenside vom Sulfonat-Typ kommen dabei vorzugsweise C₉₋₁₃-Al-kylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂₋₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete anionische Tenside.

Auch Fettalkoholethersulfate, wie die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Es ist bevorzugt, dass wenigstens eine, vorzugsweise alle erfindungsgemäßen flüssigen Zusammensetzungen eine Mischung aus Sulfonat- und Sulfat-Tensiden enthält. In einer besonders bevorzugten Ausführungsform enthält die flüssige Zusammensetzung und/oder die im Batchverfahren hergestellte Mischung C₉₋₁₃-Alkylbenzolsulfonate und Fettalkoholethersulfate als anionisches Tensid.

Zusätzlich zu dem anionischen Tensid können die flüssigen Zusammensetzungen auch Seifen enthalten. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside sowie die Seifen können in Form ihrer Natrium-, Kalium- oder Magnesium- oder Ammoniumsalze vorliegen. Vorzugsweise liegen die anionischen Tenside in Form ihrer Natriumsalze vor. Weitere bevorzugte Gegenionen für die anionischen Tenside sind auch die protonierten Formen von Cholin, Triethylamin, Monoethanolamin oder Methylethylamin. Die Zusammensetzungen können auch wenigstens ein nichtionisches Tensid aufweisen. Das nichtionische Tensid umfasst alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionisches Tensid werden vorzugsweise alkoxylierte, vorteilhafterweise ethoxylierte, insbesondere primäre Alkohole mit vorzugsweise 8 bis 18 C-Atomen und durchschnittlich 4 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 5 bis 8 EO pro Mol Alkohol bevorzugt. Zu den bevorzugten ethoxylierten Alkoholen gehören beispielsweise Ci2-i4-Alkohole mit 4 EO oder 7 EO, C₉₋₁₁-Alkohol mit 7 EO, C₁₃₋₁₅-Alkohole mit 5 EO, 7 EO oder 8 EO, C₁₂₋₁₈-Alkohole mit 5 EO oder 7 EO und Mischungen aus diesen. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das Wasch-, Reinigungs-, Nachbehandlungs- oder Waschhilfsmittel einen C₁₂₋₁₈-Fettalkohol mit 7 EO oder einen C₁₃₋₁₅-Oxoalkohol mit 7 EO als nichtionisches Tensid.

Die erfindungsgemäß hergestellten Zusammensetzungen umfassen in der Grund-Mischung weiterhin ein oder mehrere Lösungsmittel. Hierbei kann es sich um Wasser und/oder nichtwässrige Lösungsmittel handeln. Bevorzugt enthält die Mischung Wasser als Hauptlösungsmittel. Die im Batchverfahren hergestellte Vormischung kann weiterhin nichtwässrige Lösungsmittel umfassen. Geeignete nichtwässrige Lösungsmittel umfassen ein- oder mehrwertige Alkohole, Alkanolamine oder Glykolether. Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3- methoxybutanol, Propylen-glykol-t-butylether, Di-n-octylether sowie Mischungen dieser Lösungsmittel.

Weist die erfindungsgemäße Zusammensetzung ein oder mehrere auch nichtwässrige Lösungsmittel, insbesondere solche mit geringem Dampfdruck, wie beispielsweise Ethanol oder 2-Propanol, auf, werden diese vorzugsweise im kontinuierlichen Verfahren zur Mischung hinzugegeben. Im kontinuierlichen Verfahren wird in einem geschlossenen System gearbeitet, so dass das entsprechende Lösungsmittel nicht verdampfen kann. Eine Gefährdung der Umwelt wird damit reduziert und annähernd ausgeschlossen. Es ist erfindungsgemäß auch möglich, dass Wasser oder andere geeignete Lösungsmittel, unabhängig von deren Dampfdruck, im kontinuierlichen Verfahren eingebracht werden.

Die erfindungsgemäßen Zusammensetzungen können weiterhin Gerüststoffe und/oder alkalische Substanzen umfassen. Diese werden besonders bevorzugt im Batchverfahren der Vormischung oder Grundmischung hinzugegeben. Es ist jedoch auch möglich, dass diese gelöst in einem geeigneten Lösungsmittel im kontinuierlichen Verfahren zugefügt werden.

Als Gerüststoffe sind beispielsweise polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Als Gerüststoffe, die in der erfindungsgemäßen Zusammensetzung enthalten sein können, sind insbesondere auch Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche weiterhin in der erfindungsgemäßen Zusammensetzung vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Bevorzugt werden allerdings lösliche Gerüststoffe, wie beispielsweise Citronensäure, oder Acrylpolymere mit einer Molmassen von 1.000 bis 5.000 g / mol in der Grundrezeptur eingesetzt.

Alkalische Substanzen oder Waschalkalien sind im Sinne der vorliegenden Erfindung Chemikalien zur Anhebung und Stabilisierung des pH-Wertes der Zusammensetzung.

Die Grundmischung weist insbesondere Farbstoffe, Parfümzusammensetzungen, Enzyme, Parfümkapseln, Mikrobeads, Trübungsmittel, Farbtransferinhibitoren, Aufheller, Salzlösungen, Co-Tenside und Wasser oder andere Lösungsmittel auf.

Durch das Co-Tensid oder die Co-Tenside wird die micellare Struktur der Tenside in der Grundmischung verändert. Durch einen oder mehrere Elektrolyte kann diese Wirkung verstärkt werden. Hierdurch entsteht eine lamellare Struktur der Tenside. Entsprechende strukturierte Wasch- oder Reinigungsmittel mit Fließgrenze sind im Stand der Technik beispielsweise in WO 2013/064357 A1 beschrieben. Auf den Inhalt diese Anmeldung wird vollumfänglich Bezug genommen. Co-Tenside im Sinne der vorliegenden Erfindung sind amphiphile Moleküle mit kleiner, hydrophiler Kopfgruppe. In einem binären System mit Wasser sind diese Co-Tenside oftmals nur schwach oder gar nicht löslich. Entsprechend bilden sie dort auch keine Micellen aus. In Gegenwart der Tenside der Grundrezeptur werden die Co-Tenside in deren Assoziate eingebaut und verändern dadurch die Morphologie dieser Assoziate. Aus den Kugelmicellen werden Stäbchen- und/oder Scheibchenmicellen. Bei einem ausreichend hohem Gesamttensidgehalt kommt es zu der Ausbildung lamellarer Phasen beziehungsweise Strukturen.

Das Co-Tensid ist vorzugsweise ausgewählt aus der Gruppe bestehend aus alkoxylierten C₈-C₁₈-Fettalkoholen mit einem Alkoxylierungsgrad < 3, aliphatischen C₆-C₁₄-Alkoholen, aromatischen C₆-C₁₄-Alkoholen, aliphatischen C₆-C₁₂-Dialkoholen, Monoglyceride von C₁₂-C₁₈-Fettsäuren, Monoglycerinether von C₈-C₁₈-Fettalkoholen und Mischungen daraus. Weitere geeignete Co-Tenside sind 1-Hexanol, 1-Heptanol, 1-Octanol, 1 ,2-Octandiol, Stearinmonoglycerin und Mischungen daraus.

Ebenso eignen sich Duftalkohole wie beispielsweise Geraniol, Nerol, Citronellol, Linalool, Rhodinol und andere Terpenalkohole oder Duftaldehyde wie Lilial oder Decanal als Co-Tenside.

Bevorzugte Co-Tenside sind C₁₂-C₁₈-Fettalkohole mit einem Alkoxylierungsgrad ≤ 3. Diese Co-Tenside werden besonders gut in die bevorzugten Assoziate aus anionischem und nichtionischem Tensid eingebaut.

Geeignete alkoxylierte C₁₂-C₁₈-Fettalkohole mit einem Alkoxylierungsgrad ≤ 3 umfassen beispielsweise i-C₁₃H₂₇O(CH₂CH₂O)₂H, i-C₁₃H₂₇O(CH₂CH₂O)₃H, C₁₂-C₁₄-Alkohol mit 2 EO, C₁₂₋₁₄-Alkohol mit 3 EO, C₁₃₋₁₅-Alkohol mit 3 EO, C₁₂₋₁₈-Alkohole mit 2 EO und C₁₂₋₁₈-Alkohole mit 3 EO.

Ein Elektrolyt im Sinne der vorliegenden Erfindung ist ein anorganisches Salz. Bevorzugte anorganische Salze umfassen Natriumchlorid, Kaliumchlorid, Natriumsulfat, Natriumcarbonat, Kaliumsulfat, Kaliumcarbonat, Natriumhydrogencarbonat, Kaliumhydrogencarbonat, Calciumchlorid, Magnesiumchlorid und Mischungen daraus. Besonders stabile Zusammensetzungen werden bei Einsatz von Natriumchlorid oder Mischungen von Natriumchlorid und Kaliumsulfat erhalten.

Die Zugabe des anorganischen Salzes unterstützt die Ausbildung lamellarer Strukturen. Zusätzlich hat das anorganische Salz einen Einfluss auf die Viskosität, so dass mit Hilfe des anorganischen Salzes die Viskosität der flüssigen Zusammensetzung eingestellt werden kann.

Vorzugsweise wird die Fließgrenze durch die Dosierung der Co-Tenside und/oder einem oder mehrerer Elektrolyte in dem kontinuierlichen Verfahren erzeugt. Dies hat den Vorteil, dass die im kontinuierlichen Verfahren dosierten Bestandteile gleich in der gewünschten lamellaren Struktur vorliegen. Insbesondere beträgt der Anteil an Co-Tensiden und/oder Elektrolyten in der finalen flüssigen, Tensid-enthaltenden Zusammensetzung mit Fließgrenze bis zu 15 Gew-%, bevorzugt bis zu 10 Gew.-%, noch mehr bevorzugt bis zu 5 Gew.-%.

Die Viskosität der Grundmischung beträgt insbesondere 1000 mPa·s oder weniger, insbesondere 100 bis 900 mPa·s, vorzugsweise 200 bis 800 mPa s, besonders 400 bis 700 mPa s. Die Viskosität wird bei einer Temperatur von 20° C mit einem Viskosimeter HATDV II von Brookfield, 20 U/min, Spindel 2, gemessen.

Die Grundmischung kann auch weiterhin dispergierte Partikel aufweisen. Dispergierte Partikel im Sinne der vorliegenden Erfindung sind im Lösungsmittel der Grundmischung nicht löslich, sie können darin jedoch dispergiert werden. Erfindungsgemäß können diese dispergierten Partikel funktionell sein und/oder eine ästhethische Funktion haben. Funktionelle Materialien beeinflussen die Wirkung der Zusammensetzung wohingegen ästhetische Materialien lediglich das Aussehen oder den Geruch beeinflussen. Bevorzugt handelt es sich bei den dispergierten Partikeln um sichtbare Partikel. Dies bedeutet, dass die Partikel für den Verbraucher mit dem Auge in den Zusammensetzungen deutlich zu erkennen und von den übrigen Bestandteilen zu unterscheiden sind. Bevorzugt sind hiermit gefärbte Partikel gemeint. Besonders bevorzugt kann eine oder mehrere der Zusammensetzungen einen gelösten Farbstoff und zusätzlich farbige Partikel enthalten, die eine Farbe aufweisen, die eine Kontrastfarbe zu dem gelösten Farbstoff darstellt.

Funktionelle dispergierte Partikel können im Sinne der vorliegenden Erfindung Kapseln, Abrasivstoffe, Granulate oder Compounds sein. Unter dem Begriff Kapsel werden einerseits Aggregate mit einer Kern-Hülle-Struktur und andererseits Aggregate mit einer Matrix verstanden. Kern-Hülle-Kapsein (Mikrokapseln, Microbeads) enthalten mindestens einen festen oder flüssigen Kern, der von mindestens einer kontinuierlichen Hülle, insbesondere einer Hülle aus Polymer(en) umschlossen ist.

Im Inneren der Kapseln können empfindliche, chemische physikalisch inkompatible sowie flüchtige Komponenten (=Wirkstoffe) der flüssigen Zusammensetzung lager- und transportstabil eingeschlossen werden. In den Kapseln können sich beispielsweise optische Aufheller, Tenside, Komplexbildner, Bleichmittel, optische Aufheller, Bleichaktivatoren, Bleichkatalysatoren, Färb- und Duftstoffe, Antioxidantien, Gerüststoffe, Enzyme, Enzymstabilisatoren, antimikrobielle Wirkstoffe, Vergrauungsinhibitoren, Anti-Redepositionsmittel, pH-Stellmittel, Elektrolyte, Waschkraftverstärker, Vitamine, Proteine, Schauminhibitoren und/oder UV-Absorber finden. Die Füllungen der Kapseln können Feststoffe oder Flüssigkeiten in Form von Lösungen oder Emulsionen beziehungsweise Suspensionen sein. Die dispergierten Partikel können eine Dichte aufweisen, die der der flüssigen Zusammensetzung entspricht. Erfindungsgemäß bedeutet dies, dass die Dichte der dispergierten Partikel 90% bis 10% der Zusammensetzung entspricht. Es ist jedoch auch möglich, dass die dispergierten Partikel eine andere Dichte aufweisen. Dennoch ist es aufgrund des erfindungsgemäßen Verfahrens auch hier möglich, eine gleichmäßige Dispersion der Partikel in der Zusammensetzung zu erhalten. Sie können aus unterschiedlichen Materialien wie zum Beispiel Alginaten, Gelatine, Cellulose, Agar, Wachsen oder Polyethylenen bestehen. Partikel, die keine Kern-Hülle-Struktur aufweisen, können auch einen Wirkstoff in einer Matrix aus einem matrixbildenden Material aufweisen. Solche Partikel werden als "Speckles" bezeichnet. Die Matrixbildung erfolgt bei diesen Materialien beispielsweise über Gelierung, Polyanionen-Polykationen-Wechselwirkung oder Polyelektrolyt-Metallionen-Wechselwirkung und ist im Stand der Technik ebenso wie die Herstellung von Partikeln mit diesen matrixbildenden Materialien wohl bekannt.

Die erfindungsgemäße Zusammensetzung ist insbesondere ein Körperpflege-, Wasch- oder Reinigungsmittel. Körperpflege-, Wasch- oder Reinigungsmittel im Sinne der vorliegenden Erfindung umfassen Kosmetika, Haushaltsreiniger, Wäscheweichspüler, Waschmittel für Wäsche, Bodenpflegemittel, Allesreiniger, Geschirrspülmittel zur manuellen sowie maschinellen Reinigung, Vollwaschmittel, Shampoos, Duschgele und Schaumbäder, bevorzugt ist es ein Wasch- oder Reinigungsmittel.

Das erfindungsgemäße Verfahren ermöglicht somit die Herstellung von einander verschiedener Zusammensetzungen, die gleichzeitig in ein gemeinsames Behältnis abgefüllt werden können, wobei das gemeinsame Behältnis mindestens zwei Kammern aufweist, wobei die erste Zusammensetzung in eine erste Kammer, die zweite Zusammensetzung in eine davon getrennte zweite Klammer sowie jede weitere Zusammensetzung in eine weitere getrennte Kammer angefüllt wird. Das Verfahren ist vorzugsweise dadurch gekennzeichnet, dass die Grundmischung die Schritte a, b und ggf. c sowie ggf. weitere Schritte derart zyklisch durchläuft, dass die mindestens eine erste Zusammensetzung und die mindestens eine zweite Zusammensetzung sowie ggf. die mindestens eine dritte Zusammensetzung und jede weitere Zusammensetzung gleichzeitig in voneinander getrennte Kammern des gemeinsamen Behältnisses angefüllt werden.

Das erfindungsgemäße Verfahren ermöglicht damit die Herstellung von Mehrkammerbeuteln mit unterschiedlichen Zusammensetzungen in den jeweiligen Kammern, wobei die Abfüllung gleichzeitig erfolgen kann. Der Installationsaufwand ist gegenüber anderen Möglichkeiten gering und die Auslastung der Anlage kann optimiert werden.

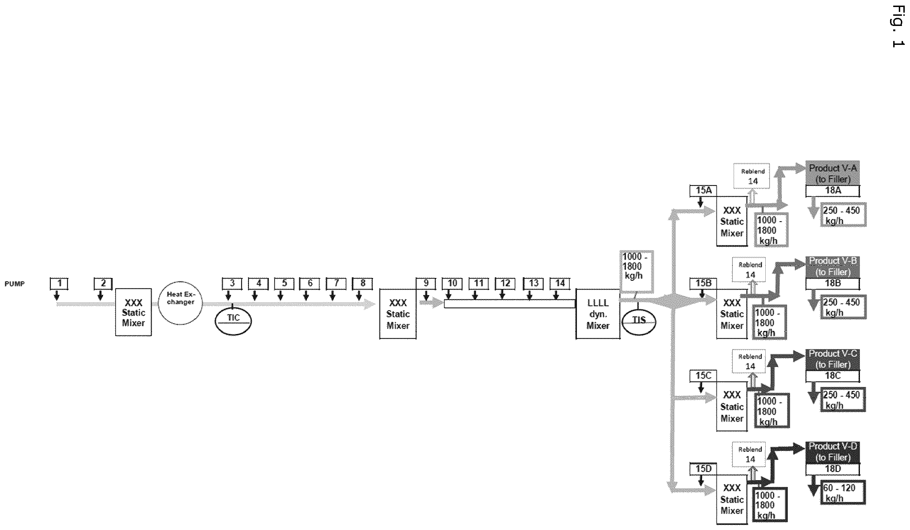

In der beigefügten Fig. 1 wird eine erfindungsgemäß bevorzugte Ausführungsform genauer erläutert. In dieser werden 4 voneinander verschiedene Zusammensetzungen hergestellt.

Dargestellt ist eine Konti-Anlage, in der über unterschiedliche Zuleitungen Bestandteile der erfindungsgemäßen Zusammensetzung in die Hauptleitung dosiert werden. Beispielshaft stehen die Bezugszeichen 1 bis 17 für die Zuleitung der folgenden Bestandteile:
- 1: Lösungsmittel (Wasser oder nichtwässriges Lösungsmittel) oder Masterbatch
- 2: Lösungsmittel (Wasser oder nichtwässriges Lösungsmittel) oder Masterbatch oder Konservierungsmittel
- 3 bis 8: Lösungsmittel, insbesondere nichtwässriges Lösungsmittel, oder Hilfsstoffe zur Einstellung der Viskosität oder des pH-Werts oder Konservierungsmittel oder Trübungsmittel oder Farbtransferinhibitoren oder Aufheller oder Salzlösungen oder Tenside oder Co-Tenside
- 9 bis 13: Hilfsstoffe zur Einstellung der Viskosität oder des pH-Werts oder Trübungsmittel oder Farbtransferinhibitoren oder Aufheller oder Salzlösungen oder Co-Tenside oder Parfum oder Enzyme
- 14: Rückführung von Mischungen aus 15A, 15B, 15C, 15D
- 15A: erstes Additiv
- 15B: zweites Additiv

- 15C: drittes Additiv
- 15D: viertes Additiv

Durch die jeweiligen Zuleitungen können erfindungsgemäß auch andere oder weitere Bestandteile in dieser oder einer anderen Reihenfolge in den Hauptstrom eingebracht werden. Dabei ist jeweils die im Hauptstrom herrschende Temperatur, die Anzahl und Position der Mischer sowie die Reihenfolge, in der die Bestandteile zugegeben werden vom Fachmann zu beachten. Erfindungsgemäß wird über jede der Zuleitungen vorzugsweise nur jeweils ein Material in die Zuleitung eingebracht. So können beispielsweise über Zuleitung 1 Wasser, über Zuleitung 2 der Masterbatch und über Zuleitung 3 Ethanol dosiert werden. Alternativ ist es auch möglich, dass über Zuleitung 1 Ethanol, über Zuleitung 2 Waser und über Zuleitung 3 der Masterbatch dosiert wird. Entsprechendes gilt für die weiteren Zuleitungen.

Die weiteren Bestandteile sind insbesondere:
- TIC: Temperaturregelung
- TIS: Temperatur-Schaltpunkt

Statischer Mischer (XXX Static Mixer)
Dynamischer Mischer (LLLL dyn. Mixer)
Wärmetauscher (Heat Exchanger)

In der in Fig. 1 beispielhaft gezeigten Ausführungsform sind unterschiedliche Zuleitungen für Enzyme (9 bis 13) gezeigt. Alle befinden sich entlang der Fließrichtung innerhalb der Hauptleitung in der zweiten Hälfte der Anlage, werden also gegen Ende des Verfahrens zugegeben. Dies hat den Vorteil, dass hier die Mischung beispielsweise durch einen Kühler und Mischer sowie gegebenenfalls die Zufuhr von vorzugsweise kaltem Wasser (1, 2, 3, 4, 5, 6, 7, 8) zur direkten Kühlung abgekühlt wird, so dass eine Degradation der Enzyme nicht mehr stattfindet. Dabei ist es erfindungsgemäß möglich nur über eine der Zuleitungen (9, 10, 11, 12, 13) Enzyme zu dosieren. Es ist erfindungsgemäß auch möglich, über mehrere Zuleitungen Enzyme in den Hauptstrom zu dosieren. Dabei können über unterschiedliche Zuleitungen gleiche oder voneinander verschiedene Enzyme dosiert werden. Unterschiedliche Enzyme können auch über gleiche Leitungen dosiert werden.

Nach der Zugabe aller wesentlicher Bestandteile, also nach Zulauf 13, wird der gesamte Strom nach einer finalen Durchmischung zunächst in Richtung des Zulaufs 15A geleitet, in welchem das wenigstens eine erste Additiv zugegeben wird. Nach einer Zeit t1, in welcher eine ausreichende Menge an der ersten Zusammensetzung produziert wurde, wird vorzugweise der Hauptstrom zunächst abgeleitet und separat gelagert. Dieser Reblend der ersten Zusammensetzung kann bei der erneuten Herstellung der ersten Zusammensetzung über Zuleitung 14 wieder in das Verfahren eingeleitet werden. Dieser Zeitraum dauert nur wenige Sekunden.

Anschließend wird der gesamte Strom in Richtung des Zulaufs 15B geleitet, in welchem das zweite Additiv zugegeben wird. Dies erfolgt für die Zeit t2. Anschließend wird der Strom in Richtung Zulauf 15C umgeleitet und dran anschließend zu Zulauf 15D. Anschließend erfolgt erneut die Umleitung des Stroms zu Zulauf 15A, so dass zyklisch nacheinander erste, zweite, dritte und vierte Zusammensetzung erhalten werden.

## Patentansprüche

1. Verfahren zur kontinuierlichen Herstellung wenigstens zwei voneinander verschiedener flüssiger, Tensid enthaltenden Zusammensetzungen, in welchem kontinuierlich ein Strom einer Grundmischung, welche wenigstens ein Tensid und wenigstens ein Lösungsmittel aufweist, bereitgestellt wird und diese Grundmischung zyklisch mindestens die folgenden Schritte durchläuft:
a. versetzen des Stroms der Grundmischung für eine erste Zeitdauer mit mindestens einem ersten Additiv, um eine erste Zusammensetzung zu erhalten,
und
b. versetzen des Stroms der Grundmischung für eine zweite Zeitdauer mit mindestens einem zweiten Additiv, um eine zweite Zusammensetzung zu erhalten,
wobei das mindestens eine erste Additiv und das mindestens eine zweite Additiv und gegebenenfalls das mindestens eine dritte und jede weitere Additiv jeweils wenigstens einen Farbstoff aufweisen, wobei die Farbstoffe voneinander verschieden sind und die erste und zweite Zusammensetzung getrennt gelagert werden.

2. Verfahren zur kontinuierlichen Herstellung wenigstens zwei voneinander verschiedener flüssiger, Tensid enthaltenden Zusammensetzungen, in welchem kontinuierlich ein Strom einer Grundmischung, welche wenigstens ein Tensid und wenigstens ein Lösungsmittel aufweist, bereitgestellt wird und diese Grundmischung zyklisch mindestens die folgenden Schritte durchläuft:
a. versetzen des Stroms der Grundmischung für eine erste Zeitdauer mit mindestens einem ersten Additiv, um eine erste Zusammensetzung zu erhalten,
und
b. versetzen des Stroms der Grundmischung für eine zweite Zeitdauer mit mindestens einem zweiten Additiv, um eine zweite Zusammensetzung zu erhalten,
wobei das mindestens eine zweite Additiv und/oder gegebenenfalls das mindestens eine dritte und jedes weitere Additiv ein oder mehrere Enzyme aufweisen, wobei die Enzyme voneinander verschieden sind und die erste und zweite Zusammensetzung getrennt gelagert werden.

3. Verfahren zur kontinuierlichen Herstellung wenigstens zwei voneinander verschiedener flüssiger, Tensid enthaltenden Zusammensetzungen, in welchem kontinuierlich ein Strom einer Grundmischung, welche wenigstens ein Tensid und wenigstens ein Lösungsmittel aufweist, bereitgestellt wird und diese Grundmischung zyklisch mindestens die folgenden Schritte durchläuft:
a. versetzen des Stroms der Grundmischung für eine erste Zeitdauer mit mindestens einem ersten Additiv, um eine erste Zusammensetzung zu erhalten,
und
b. versetzen des Stroms der Grundmischung für eine zweite Zeitdauer mit mindestens einem zweiten Additiv, um eine zweite Zusammensetzung zu erhalten,
wobei das mindestens eine erste Additiv und/oder das mindestens eine zweite Additiv und/oder gegebenenfalls das mindestens eine dritte und jedes weitere Additiv mindestens einen optischen Aufheller enthält, wobei ein Additiv entweder mindestens ein Enzym oder mindestens einen optischen Aufheller aufweist und die erste und zweite Zusammensetzung getrennt gelagert werden.

4. Verfahren nach einem der Ansprüche 1 bis 3, **dadurch gekennzeichnet, dass** die Grundmischung zyklisch mindestens die folgenden Schritte durchläuft:
a. versetzen des Stroms der Grundmischung für eine erste Zeitdauer mit mindestens einem ersten Additiv, um eine erste Zusammensetzung zu erhalten,
und
b. versetzen des Stroms der Grundmischung für eine zweite Zeitdauer mit mindestens einem zweiten Additiv, um eine zweite Zusammensetzung zu erhalten,
c. versetzen des Stroms der Grundmischung für eine dritte Zeitdauer mit mindestens einem dritten Additiv, um eine dritte Zusammensetzung zu erhalten,
wobei das mindestens eine erste Additiv und das mindestens eine zweite Additiv und das mindestens eine dritte Additiv voneinander verschieden sind.

5. Verfahren nach einem der Ansprüche 1 bis 4, **dadurch gekennzeichnet, dass** die Grundmischung in einem kontinuierlichen Verfahren oder einem Batch-Verfahren hergestellt wird.

6. Verfahren nach einem der Ansprüche 1 bis 4, **dadurch gekennzeichnet, dass** die Grundmischung dadurch bereitgestellt wird, dass in einem Batch-Verfahren eine Vormischung, die wenigstens ein Tensid und wenigstens ein Lösungsmittel aufweist, hergestellt wird, zu welcher dann in einem kontinuierlichen Verfahren wenigstens eine weitere Komponente zugegeben wird.

7. Verfahren nach Anspruch 6, **dadurch gekennzeichnet, dass** der Anteil an allen Bestandteilen der Zusammensetzungen, die im Batchverfahren hergestellt werden, 1 Vol.-% bis 99 Vol.-%, 5 Vol.-% bis 95 Vol.-%, noch mehr bevorzugt insbesondere 20 Vol.-% bis 90 Vol.-% beträgt, bezogen auf das Gesamtvolumen der Grundmischung.

8. Verfahren nach Anspruch 6 oder 7, **dadurch gekennzeichnet, dass** der Anteil an allen Bestandteilen der Zusammensetzungen, die im kontinuierlichen Verfahren hergestellt werden, 1 Vol.-% bis 99 Vol.-%, insbesondere 5 Vol.-% bis 95 Vol.-%, noch mehr bevorzugt 10 Vol.-% bis 80 Vol.-% beträgt, bezogen auf das Gesamtvolumen der Grundmischung.

9. Verfahren nach einem der Ansprüche 6 bis 8, **dadurch gekennzeichnet, dass** die Vormischung Aniontenside in einem Anteil von 5 Gew.-% bis 40 Gew.-%, insbesondere von 8 Gew.-% bis 36 Gew.-%, besonders von 10 Gew.-% bis 30 Gew.-% aufweist, bezogen auf das Gesamtgewicht der Vormischung.

10. Verfahren nach einem der Ansprüche 6 bis 9, **dadurch gekennzeichnet, dass** Vormischung Niotenside in einem Anteil von 1 Gew.-% bis 27 Gew.-%, insbesondere von 3 Gew.-% bis 25 Gew.- %, besonders von 5 Gew.-% bis 20 Gew.-% aufweist, bezogen auf das Gesamtgewicht der Vormischung.

11. Verfahren nach einem der Ansprüche 1 bis 10, **dadurch gekennzeichnet, dass** die hergestellten verschiedenen Zusammensetzungen in ein gemeinsames Behältnis, welches für jede Zusammensetzung eine eigene Kammer aufweist, abgefüllt werden.

12. Verfahren nach Anspruch 11, **dadurch gekennzeichnet, dass** die Grundmischung die Schritte a., b. und gegebenenfalls c. derart zyklisch durchläuft, dass die mindestens eine erste Zusammensetzung und die mindestens eine zweite Zusammensetzung sowie gegebenenfalls die mindestens eine dritte Zusammensetzung und jede weitere Zusammensetzung gleichzeitig in voneinander getrennte Kammern des gemeinsamen Behältnisses abgefüllt werden.

13. Verfahren nach einem der Ansprüche 1 bis 12, **dadurch gekennzeichnet, dass** die Tensid enthaltenden Zusammensetzungen Körperpflege-, Wasch- oder Reinigungsmittel sind, insbesondere Wasch- oder Reinigungsmittel.

## Claims

1. Process for the continuous preparation of at least two mutually different liquid surfactant-containing compositions, in which a stream of a base mixture comprising at least one surfactant and at least one solvent is continuously provided and this base mixture cyclically passes through at least the following steps:
a. adding at least a first additive to the stream of base mixture for a first period of time to obtain a first composition,
and
b. adding at least a second additive to the stream of the base mixture for a second period of time to obtain a second composition,
wherein the at least one first additive and the at least one second additive and optionally the at least one third and each further additive each comprise at least one dye, wherein the dyes are different from each other and the first and second compositions are stored separately.

2. Process for the continuous preparation of at least two mutually different liquid surfactant-containing compositions, in which a stream of a base mixture comprising at least one surfactant and at least one solvent is continuously provided and this base mixture cyclically passes through at least the following steps:
a. adding at least a first additive to the stream of base mixture for a first period of time to obtain a first composition,
and
b. adding at least a second additive to the stream of the base mixture for a second period of time to obtain a second composition,
wherein the at least one second additive and/or optionally the at least one third additive and any further additive comprise one or more enzymes, wherein the enzymes are different from each other and the first and second compositions are stored separately.

3. Process for the continuous preparation of at least two mutually different liquid surfactant-containing compositions, in which a stream of a base mixture comprising at least one surfactant and at least one solvent is continuously provided and this base mixture cyclically passes through at least the following steps:
a. adding at least a first additive to the stream of base mixture for a first period of time to obtain a first composition,
and
b. adding at least a second additive to the stream of the base mixture for a second period of time to obtain a second composition,
wherein the at least one first additive and/or the at least one second additive and/or optionally the at least one third and each further additive comprises at least one optical brightener, wherein an additive comprises either at least one enzyme or at least one optical brightener, and the first and second compositions are stored separately.

4. Process according to any one of claims 1 to 3, **characterized in that** the base mixture cyclically passes through at least the following steps:
a. adding at least a first additive to the stream of base mixture for a first period of time to obtain a first composition,
and
b. adding at least a second additive to the stream of the base mixture for a second period of time to obtain a second composition,
c. adding at least a third additive to the base mixture stream for a third period of time to obtain a third composition,
wherein the at least one first additive and the at least one second additive and the at least one third additive are different from each other.

5. Process according to any one of claims 1 to 4, **characterized in that** the base mixture is prepared in a continuous process or a batch process.

6. Process according to any one of claims 1 to 4, **characterized in that** the base mixture is provided by preparing in a batch process a premix comprising at least one surfactant and at least one solvent, to which at least one further component is then added in a continuous process.

7. Process according to claim 6, **characterized in that** the proportion of all constituents of the compositions prepared by batch process is 1% by volume to 99% by volume, 5% by volume to 95% by volume, even more preferably in particular 20% by volume to 90% by volume, based on the total volume of the base mixture.

8. Process according to claim 6 or 7, **characterized in that** the proportion of all constituents of the compositions prepared in the continuous process is from 1% to 99% by volume, in particular from 5% to 95% by volume, even more preferably from 10% to 80% by volume, based on the total volume of the base mixture.

9. Process according to any one of claims 6 to 8, **characterized in that** the premix comprises anionic surfactants in a proportion of from 5% by weight to 40% by weight, in particular from 8% by weight to 36% by weight, especially from 10% by weight to 30% by weight, based on the total weight of the premix.

10. Process according to any one of claims 6 to 9, **characterized in that** premix has niosurfactants in a proportion of from 1% by weight to 27% by weight, in particular from 3% by weight to 25% by weight, especially from 5% by weight to 20% by weight, based on the total weight of the premix.

11. Process according to any one of claims 1 to 10, **characterized in that** the different compositions prepared are filled into a common container having a separate chamber for each composition.

12. Process according to claim 11, **characterized in that** the base mixture is cyclically passed through steps a., b. and optionally c. in such a way that the at least one first composition and the at least one second composition, and optionally the at least one third composition and each further composition, are simultaneously filled into chambers of the common container which are separated from one another.

13. Process according to any one of claims 1 to 12, **characterized in that** the surfactant-containing compositions are personal care, detergent or cleaning compositions, in particular washing or cleaning compositions.

## Revendications

1. Procédé de préparation en continu d'au moins deux compositions liquides différentes l'une de l'autre, contenant un agent tensioactif, dans lequel on prépare en continu un flux d'un mélange de base, qui présente au moins un agent tensioactif et au moins un solvant, et ce mélange de base passe de manière cyclique au moins par les étapes suivantes:
a. ajouter au flux du mélange de base, pendant une première période de temps, au moins un premier additif pour obtenir une première composition,
et
b. ajouter au flux du mélange de base, pendant une deuxième période de temps, au moins un deuxième additif pour obtenir une deuxième composition,
dans laquelle ledit au moins un premier additif et ledit au moins un deuxième additif, et éventuellement ledit au moins un troisième additif et chaque additif supplémentaire, comprennent chacun au moins un colorant, lesdits colorants étant différents les uns des autres, et lesdites première et deuxième compositions étant stockées séparément.

2. Procédé de préparation en continu d'au moins deux compositions liquides différentes l'une de l'autre, contenant un agent tensioactif, dans lequel on prépare en continu un flux d'un mélange de base, qui présente au moins un agent tensioactif et au moins un solvant, et ce mélange de base passe cycliquement par au moins les étapes suivantes:
a. ajouter au flux du mélange de base, pendant une première période de temps, au moins un premier additif pour obtenir une première composition,
et
b. ajouter au flux du mélange de base, pendant une deuxième période de temps, au moins un deuxième additif pour obtenir une deuxième composition,
dans laquelle ledit au moins un deuxième additif et/ou éventuellement ledit au moins un troisième additif et chaque additif supplémentaire comprennent une ou plusieurs enzymes, lesdites enzymes étant différentes les unes des autres et lesdites première et deuxième compositions étant stockées séparément.

3. Procédé de préparation en continu d'au moins deux compositions liquides différentes l'une de l'autre, contenant un agent tensioactif, dans lequel on prépare en continu un flux d'un mélange de base, qui présente au moins un agent tensioactif et au moins un solvant, et ce mélange de base passe cycliquement par au moins les étapes suivantes:
a. ajouter au flux du mélange de base, pendant une première période de temps, au moins un premier additif pour obtenir une première composition,
et
b. ajouter au flux du mélange de base, pendant une deuxième période de temps, au moins un deuxième additif pour obtenir une deuxième composition,
dans laquelle ledit au moins un premier additif et/ou ledit au moins un deuxième additif et/ou éventuellement ledit au moins un troisième additif et chaque additif supplémentaire contient au moins un azurant optique, dans laquelle un additif comprend soit au moins une enzyme soit au moins un azurant optique, et la première et la deuxième composition sont stockées séparément.

4. Procédé selon l'une quelconque des revendications 1 à 3, **caractérisé en ce que** le mélange de base subit de manière cyclique au moins les étapes suivantes:
a. ajouter au flux du mélange de base, pendant une première période de temps, au moins un premier additif pour obtenir une première composition,
et
b. ajouter au flux du mélange de base, pendant une deuxième période de temps, au moins un deuxième additif pour obtenir une deuxième composition,
c. ajouter au flux du mélange de base, pendant une troisième période de temps, au moins un troisième additif pour obtenir une troisième composition,
dans lequel ledit au moins un premier additif et ledit au moins un deuxième additif et ledit au moins un troisième additif sont différents les uns des autres.

5. Procédé selon l'une quelconque des revendications 1 à 4, **caractérisé en ce que** le mélange de base est préparé selon un procédé continu ou un procédé discontinu.

6. Procédé selon l'une des revendications 1 à 4, **caractérisé en ce que** le mélange de base est préparé en produisant, dans un procédé discontinu, un prémélange qui présente au moins un agent tensioactif et au moins un solvant, auquel on ajoute ensuite, dans un procédé continu, au moins un autre composant.

7. Procédé selon la revendication 6, **caractérisé en ce que** la proportion de tous les constituants des compositions préparées par le procédé discontinu est de 1 % en volume à 99 % en volume, de 5 % en volume à 95 % en volume, de manière encore plus préférée notamment de 20 % en volume à 90 % en volume, par rapport au volume total du mélange de base.

8. Procédé selon la revendication 6 ou 7, **caractérisé en ce que** la proportion de tous les constituants des compositions qui sont préparées dans le procédé continu est de 1 % en volume à 99 % en volume, en particulier de 5 % en volume à 95 % en volume, de manière encore plus préférée de 10 % en volume à 80 % en volume, par rapport au volume total du mélange de base.

9. Procédé selon l'une des revendications 6 à 8, **caractérisé en ce que** le prémélange présente des tensioactifs anioniques dans une proportion de 5 % en poids à 40 % en poids, en particulier de 8 % en poids à 36 % en poids, en particulier de 10 % en poids à 30 % en poids, par rapport au poids total du prémélange.

10. Procédé selon l'une des revendications 6 à 9, **caractérisé en ce que** le prémélange présente des tensioactifs niotiques dans une proportion de 1 % en poids à 27 % en poids, en particulier de 3 % en poids à 25 % en poids, en particulier de 5 % en poids à 20 % en poids, par rapport au poids total du prémélange.

11. Procédé selon l'une quelconque des revendications 1 à 10, **caractérisé en ce que** les différentes compositions préparées sont conditionnées dans un récipient commun comportant un compartiment distinct pour chaque composition.

12. Procédé selon la revendication 11, **caractérisé en ce que** le mélange de base passe par les étapes a., b. et éventuellement c. de manière cyclique de telle sorte que la au moins une première composition et la au moins une deuxième composition ainsi qu'éventuellement la au moins une troisième composition et chaque autre composition sont remplies simultanément dans des compartiments séparés les uns des autres du récipient commun.

13. Procédé selon l'une des revendications 1 à 12, **caractérisé en ce que** les compositions contenant le tensioactif sont des produits de soins corporels, des produits de lavage ou de nettoyage, en particulier des produits de lavage ou de nettoyage.
